# EUROPEAN PATENT SPECIFICATION

(11) **EP 0 972 015 B1**
(45) Date of publication and mention of the grant of the patent: **07.06.2006**
(21) Application number: 97945109.3
(22) Date of filing: 06.10.1997
(51) Int. Cl.: C12N 9/14, C12N 15/55, A61K 38/46, G01N 33/573, C07K 16/40

(54) **ENZYME HAVING S-ADENOSYL-L-HOMOCYSTEINE HYDROLASE (AHCY) TYPE ACTIVITY**
ENZYME MIT S-ADENOSYL-L-HOMOCYSTEIN-HYDROLASE-ÄHNLICHER AKTIVITÄT.
ENZYME AYANT UNE ACTIVITE DE TYPE S-ADENOSYL-L-HOMOCYSTEINE HYDROLASE (AHCY)

(30) Priority: 04.10.1996 NZ 29950796
(43) Date of publication of application: 19.01.2000
(73) Proprietor: Hart, Derek Nigel John, Christchurch 4 (NZ)
(72) Inventor: Hart, Derek Nigel John, Christchurch 4 (NZ)
(74) Representative: Cornish, Kristina Victoria Joy
(86) International application number: PCT/NZ1997/000133
(87) International publication number: WO 1998/014562

(56) References cited:
- COULTER-KARIS D. E. AND HERSHFIELD M. S.: "Sequence of full length cDNA for human S-adenosylhomocysteine hydrolase" ANNALS OF HUMAN GENETICS, vol. 53, May 1989 (1989-05), pages 169-175, XP001068806
- MERTA A ET AL: "THE GENE AND PSEUDOGENES OF RAT S-ADENOSYL-L-HOMOCYSTEINE HYDROLASE" EUROPEAN JOURNAL OF BIOCHEMISTRY, BERLIN, DE, vol. 229, no. 2, 15 April 1995 (1995-04-15), pages 575-582, XP000601433 ISSN: 0014-2956
- ARREDONDO-VEGA F X ET AL.: "Isozyme and DNA analysis of human S-adenosyl-L-homocysteine hydrolase (AHCY)" ANNALS OF HUMAN GENETICS, vol. 53, May 1989 (1989-05), pages 157-167, XP001068879
- BISSBORT S ET AL.: "Genetics of human S-adenosylhomocysteine hydrolase. A new polymorphism in man." HUMAN GENETICS., vol. 65, no. 1, 1983, pages 68-71, XP001068885
- PILZ A. ET AL.: "Mapping of the structural gene for S-adenosyl homocysteine hydrolase to mouse chromosome 2, and related sequences to chromosome 8 and X " MAMMALIAN GENOME, vol. 3, no. 11, 1992, pages 633-636, XP001070122
- DEKKER J W ET AL.: "Identification of an S-adenosylhomocysteine hydrolase-like transcript induced during dendritic cell differenciation" IMMUNOGENETICS, vol. 53, 13 February 2002 (2002-02-13), pages 993-1001, XP001068467
- PROC. NATL. ACAD. SCI. U.S.A., Vol. 84, No. 719, OGAWA H. et al., "Amino Acid Sequence of S-Adenosyl-L-Homocysteine Hydrolase from Rat Liver", pages 719-723. XP001068976
- PROC. NATL. ACAD. SCI. U.S.A., Vol. 92, 1995, MARTIN C.H. et al., "Complete Sequence of the Bithorax Complex of Drosphilia", pages 8392-8402. XP001068979
- BIOCHEM. AND BIOPHYS. RES. COM., Vol. 153, No. 1, KASHIR J. et al., "Amino Acid Sequence of S-Adenosyl-L-Homocysteine Hydrolase from DICTYOSTELIUM DISCOIDEUM as Deduced from the cDNA Sequence", pages 359-364. XP001069972
- J. BIOL. CHEM., Vol. 261, No. 29, 1986, GOMI T. et al., "S-Adenosyl Homocysteinase from Rat Liver", pages 13422-13425. XP001069971

## Description

This invention relates to a novel enzyme of the S-adenosyl-L-homocysteine hydrolase type and to methods which employ this enzyme.

### BACKGROUND

S-adenosyl-L-homocysteine hydrolase (AHCY) is an enzyme which is involved in the pathway of S-adenosylmethionine metabolism. S-adenosylmethionine (AdoMet) plays a pivotal role as a methyl donor in a myriad of biological and biochemical events. For example, AdoMet donates methyl groups to small molecules like norepinephrine, yielding the potent product epinephrine; to medium sized molecules such as phosphatidyl ethanolamine, eventually formingphosphatidylcholine in the lipid bilayer; and to RNA and DNA, thereby modulating transcription and translation processes. AdoMet - dependent methylation reactions have been postulated to regulate biological phenomena by post-translational modification as seen in the carboxymethylation of proteins and perhaps membrane functions via methylation of membrane phospholipids¹⁶.

S-adenosyl homocysteine (AdoHcy), formed after donation of the methyl group of AdoMet to a methyl acceptor is then hydrolysed to adenosine (Ado) and homocysteine (Hcy) by AdoHcy hydrolase (AHCY). Ado can either be deaminated by Ado deaminase (ADA) to inosine or be phosphorylated by Ado deaminase to become AMP.

Inhibition of AHCY results in an increase in intracellular levels of AdoHcy which is a potent inhibitor of AdoMet dependent methylation reactions²⁻⁴. AdoHcy has also been reported to be a competitive inhibitor of PI Kinases⁵. PI kinase is required for PI metabolism and is involved in receptor mediated increases in intracellular calcium and the transduction of biochemical signals through cell membranes.

The applicants have now identified a novel enzyme of the AHCY type. It is towards this enzyme, DNA which encodes it, and methods which employ it that the present invention is *inter alia* broadly directed.

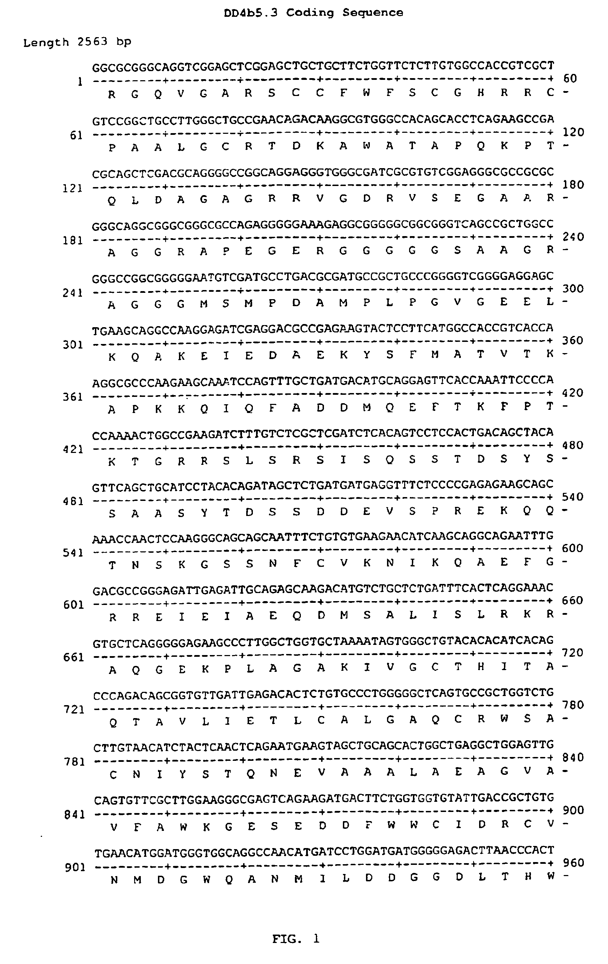

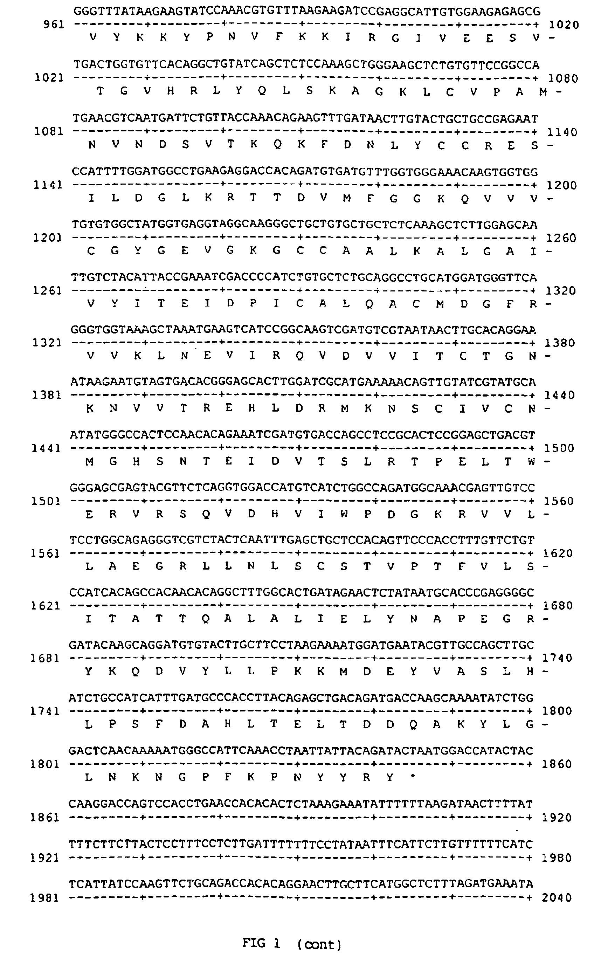

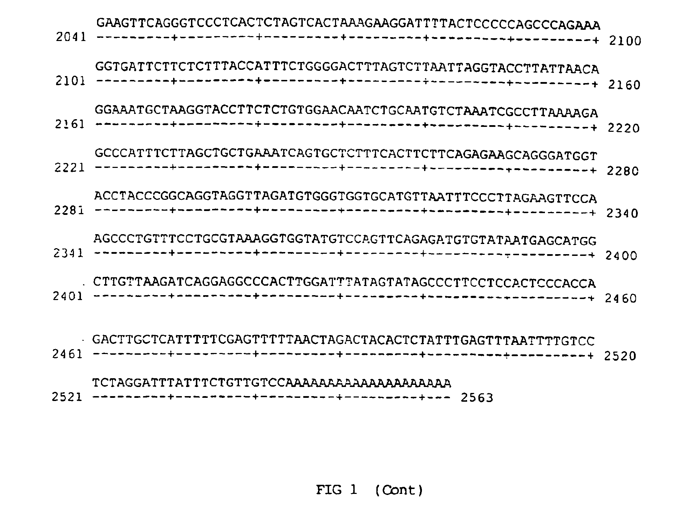

Therefore, in a first aspect, the present invention provides an enzyme having AHCY-type activity which includes amino acids 177 to 314 of the amino acid sequence of Figure 1.

Conveniently, the enzyme comprises amino acids 183 to 614 or 1 to 614 of the amino acid sequence of Figure 1.

In a further aspect, the present invention provides a DNA sequence which encodes the AHCY-type enzyme above, said sequence selected from the group of:
(a) a sequence which encodes an enzyme as defined above;
(b) a sequence which is a complement of a sequence (a);
(c) a sequence which is a reverse complement of a sequence (a); and
(d) a sequence which is a reverse sequence of a sequence (a).

Preferably, sequence (a) comprises nucleotides 529 to 945 of the Figure 1 sequence, nucleotides 549 to 1844 of the Figure 1 sequence, or nucleotides 1 to 1844 of the Figure 1 sequence.

In yet a further aspect, the invention provides DNA constructs which comprise a DNA sequence as defined above.

In still a further aspect, the invention provides a DNA construct as defined above for use in modulating the activity of an AHCY-type enzyme as defined above.

In yet a further aspect, the invention provides a method of determining the modulatory potential of a test substance (particularly a compound), which method comprises the step of determining the ability of said substance to modulate the activity of the AHCY-type enzyme defined above.

Most conveniently, the modulatory potential which will be tested for will be the ability of the test substance to inhibit the AHCY-type enzyme defined above. In this way, immunosuppressant substances can be identified.

In still further aspects, the invention provides antibodies specific for the enzyme of the invention (based upon the DNA sequence defined above). Such antibodies have utility in the identification of abnormal HD cells in cytological preparations and tissue sections, and by in situ hybridisation, respectively.

### DESCRIPTION OF THE DRAWINGS

Although the present invention is broadly as described above, it will be appreciated that it also includes embodiments described below. In particular, a person skilled in the art will appreciate that the invention will be better understood by reference to the accompanying drawing in which:
Figure 1 shows the cDNA and translated putative amino acid sequence of DD4b5.3. There is an open reading frame which extends without a stop codon for the full 5' nucleotide sequence. The initiation codon has yet to be identified:
Figure 2 shows the alignment of DD4b5.3 AHCY domain with the full length AHCY amino acid sequences of human (hm), mouse (mu) and drosophila (dr). Some conserved features shown to be important for AHCY function are indicated;
Figure 3 provides a summary of the features of the DD4b5.3 sequence:
Figure 4 shows the results of RT-PCR analysis of DD4b5.3 mRNA expression in different leucocyte populations. A Southern blot of the products probed with a specific internal probe is shown:
Figure 5 shows the results of RT-PCR analysis of different purified DC populations to examine their expression of DD4b5.3 mRNA. A semiquantitative analysis (ethidium stain) at different cycle number is shown;
Figure 6 shows the metabolic cycle of AdoMet and Ado Hey; and
Figure 7 summarises the RAP-PCR protocol.

### DESCRIPTION OF THE INVENTION

As a part of their general investigations into dendritic cells, the applicants have identified a gene encoding an enzyme of the AHCY type. The sequence of the identified gene has been found to have common features with the gene sequence of AHCY itself.

The applicants have also determined that this gene has a restricted expression pattern, being found in fresh blood dendritic cells and cultured blood dendritic cells, but not in T-cell or B-cell lymphocytes. It is therefore believed that the enzyme this gene encodes is substantially dendritic-cell-restricted. Due to the central role dendritic cells play in initiation of the primary immune response to foreign antigen, manipulation of the activity of this enzyme within dendritic cells (especially inhibition) will have a direct effect on the immune response of the host. This represents the primary, although not sole, application of this invention.

In its first aspect, the invention therefore provides the enzyme itself. As indicated above, the enzyme of the invention has AHCY-type activity and includes amino acids 177 to 314 of the amino acid sequence of Figure 1: more preferably amino acids 183 to 614 of the amino acid sequence of Figure 1: and most preferably amino acids I to 614 of the amino acid sequence of Figure 1.

The enzyme of the invention will most conveniently be produced using recombinant techniques. Therefore, in a further embodiment, the present invention provides isolated complete or partial DNA sequences encoding, or partially encoding the enzyme of the invention. Specifically, the present invention provides isolated DNA sequences comprising nucleotides 529 to 945; 549 to 1844; or 1 to 1844 of the Figure 1 sequence. Complements of such isolated DNA sequences, reverse complements of such isolated DNA sequences and reverse sequences of such isolated DNA sequences together with variants of such sequences, are also provided. DNA sequences encompassed by the present invention include cDNA, genomic DNA, recombinant DNA and wholly or partially chemically synthesized DNA molecules.

The definition of the terms "complement", "reverse complement" and "reverse sequence". as used herein, is best illustrated by the following example. For the sequence 5' AGGACC 3', the complement, reverse complement and reverse sequence are as follows:

| | |
|---|---|
| complement | 3' TCCTGG 5' |
| reverse complement | 3' GGTCCT 5' |
| reverse sequence | 5' CCAGGA 3'. |

As used herein, the term "variant" covers any sequence which exhibits at least about 70% and, more preferably, at least about 90% identity to a sequence of the present invention. Most preferably, a "variant" is any sequence which has at least about a 99% probability of being the same as the inventive sequence. The probability for DNA sequences is measured by the computer algorithm FASTA (version 2.0u4. February 1996; Pearson W. R. et al., Proc. Natl. Acad. Sci., 85:2444-2448, 1988).

The DNA sequences may be isolated by high throughput sequencing of cDNA libraries. Alternatively, oligonucleotide probes based on the sequences provided in SEQ ID NOS. 4-6 can be synthesized and used to identify positive clones in either cDNA or genomic DNA libraries by means of hybridization or PCR techniques. Probes should be at least about 10, preferably at least about 15 and most preferably at least about 20 nucleotides in length. Hybridization and PCR techniques suitable for use with such oligonucleotide probes are well known in the art. Positive clones may be analyzed by restriction enzyme digestion. DNA sequencing or the like.

In addition, the DNA sequences of the present invention may be generated by synthetic means using techniques well known in the art. Equipment for automated synthesis of oligonucleotides is commercially available from suppliers such as Perkin Elmer/Applied Biosystems Division (Foster City, CA) and may be operated according to the manufacturer's instructions.

For applications where amplification of enzyme activity is desired, the open reading frame is inserted in the DNA construct in a sense orientation, such that transformation with the DNA construct will lead to an increase in the number of copies of the gene and therefore an increase in the amount of enzyme. When down-regulation of activity is desired, the open reading frame is inserted in the DNA construct in an antisense orientation, such that the RNA produced by transcription of the DNA sequence is complementary to the endogenous mRNA sequence. This, in turn, will result in a decrease in the number of copies of the gene and therefore a decrease in the amount of enzyme. Alternatively, regulation can be achieved by inserting appropriate sequences or subsequences (e.g. DNA or RNA) in ribozyme constructs.

The DNA constructs of the present invention further comprise a gene promoter sequence and a gene termination sequence, operably linked to the DNA sequence to be transcribed, which control expression of the gene- The gene promoter sequence is generally positioned at the 5' end of the DNA sequence to be transcribed, and is employed to initiate transcription of the DNA sequence. Gene promoter sequences are generally found in the 5' non-coding region of a gene but they may exist in introns (Luehrsen. K. R, Mol. Gen. Genet. 225:81-93. 1991) or in the coding region. When the construct includes an open reading frame in a sense orientation, the gene promoter sequence also initiates translation of the open reading frame. For DNA constructs comprising either an open reading frame in an antisense orientation or a non-coding-region, the gene promoter sequence consists only of a transcription initiation site having a RNA polymerase binding site.

A variety of gene promoter sequences which may be usefully employed in the DNA constructs of the present invention are well known in the art. The promoter gene sequence, and also the gene termination sequence, may be endogenous or may be exogenous, provided the promoter is functional in the host. Preferably, gene promoter and termination sequences are from the inventive sequences themselves.

The gene termination sequence, which is located 3' to the DNA sequence to be transcribed, may come from the same gene as the gene promoter sequence or may be from a different gene. Many gene termination sequences known in the art may be usefully employed in the present invention. However, preferred gene terminator sequences are those from the original enzyme gene.

Techniques for operatively linking the components of the DNA constructs are well known in the art and include the use of synthetic linkers containing one or more restriction endonuclease sites as described, for example, by Maniatis et al., (*Molecular Cloning: A Laboratory Manual*, Cold Spring Harbor Laboratories, Cold Spring Harbor, NY, 1989). The DNA construct of the present invention may be linked to a vector having at least one replication system, for example, *E. coli,* whereby after each manipulation, the resulting construct can be cloned and sequenced and the correctness of the manipulation determined.

Having described the enzyme and the DNA constructs the present invention will now be illustrated by reference to the following non-limiting examples.

### Example 1

### (a) Identification of enzyme-encoding gene

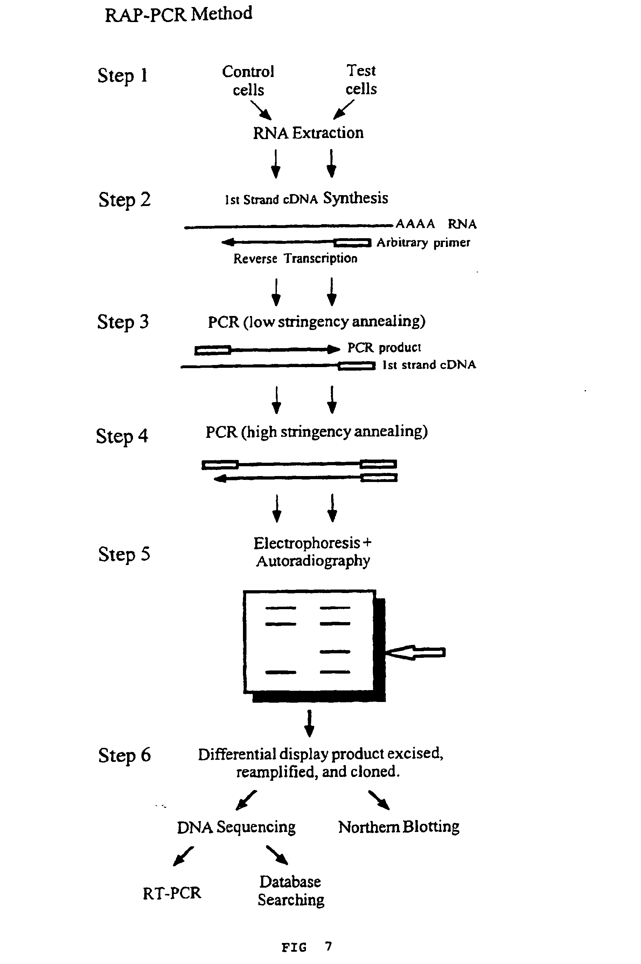

The Differential Display (DD) technique RAP-PCR was employed, using Hodgkins cell line L428 and monocytoid cell line U937. The RAP-PCR protocol is as shown in Figure 7, and was performed as follows:
Total RNA extracted from L428 and U937 cell lines (Step 1) was used for first stand cDNA was synthesis using an arbitrary oligonucleotide primer (Step 2). An aliquot of the RT reaction was used in a subsequent PCR with the same arbitrary primer in the presence of 32P-labelled dCTP. The first five cycles of the PCR was performed at the low annealing temperature of 40oC (low stringency) to allow mismatches between the arbitrary primer and cDNA template (Step 3), followed by 30 cycles at 60oC annealing temperature (high stringency) to amplify specifically the products which have incorporated the arbitrary primer (Step 4). The PCR products were electrophoresed non-denaturing sequencing gels and subjected to autoradiography. PCR products on the L428 lanes but not U937 lanes were excised from the gel using the autoradiogram as a guide (Step 5), and used as template to reamplify the products with the arbitrary primer (Step 6). Reamplified L428 specific bands were cloned in pBluescript and DNA sequenced.

One clone, called DD4b.5.3. contained a 416 bp fragment encoding part of the enzyme of the invention.

### (b) Isolation of a full length cDNA for DD4b5.3

The DD4b5.3 cDNA fragment was used to probe a Lambda ZAP express (Stratagene) L428 cDNA library produced in this laboratory. A near full length clone of 2.2 kb was identified (6.1222(1)) which was sequenced using a LI-COR automated sequencer (Figure 1, nucleotides 241-2563).

Rapid amplification of cDNA ends (RACE) was used to identify further 5' cDNA sequence using L428 cDNA as a template. RACE products were cloned and sequenced revealing further 5' sequence. Subsequently a 2nd cDNA clone (2.11(1)b) was isolated from the L-428 library. This provided further 5' sequence (Figure 1; nucleotides 1-240) with two bases of identical overlapping sequence with the first clone.

Comparison of the nucleic acid sequence revealed a significant portion of identity with the cDNA sequence for an enzyme called L-adenosine-S homocysteine hydrolase (AHCY).

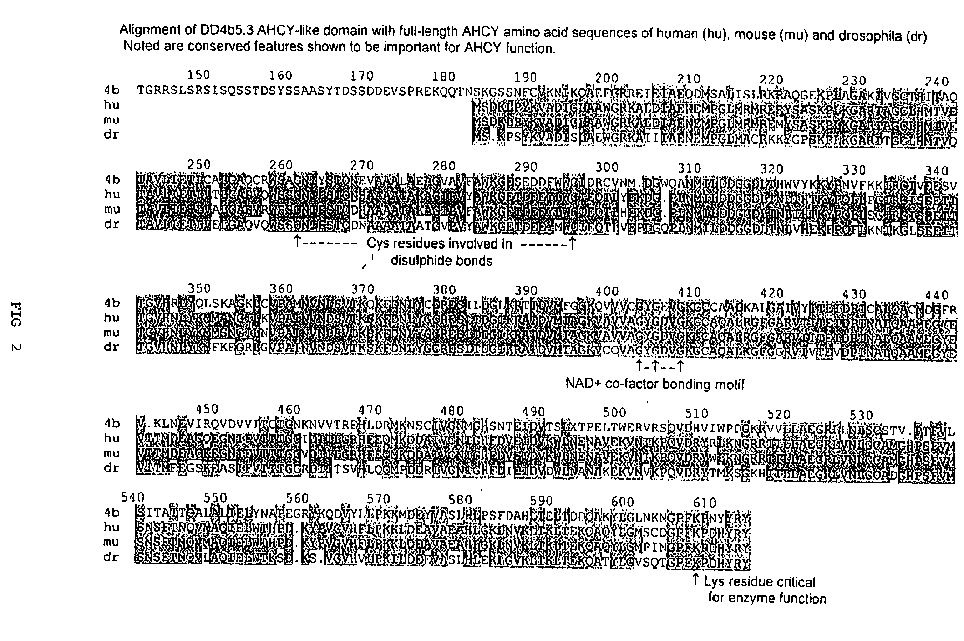

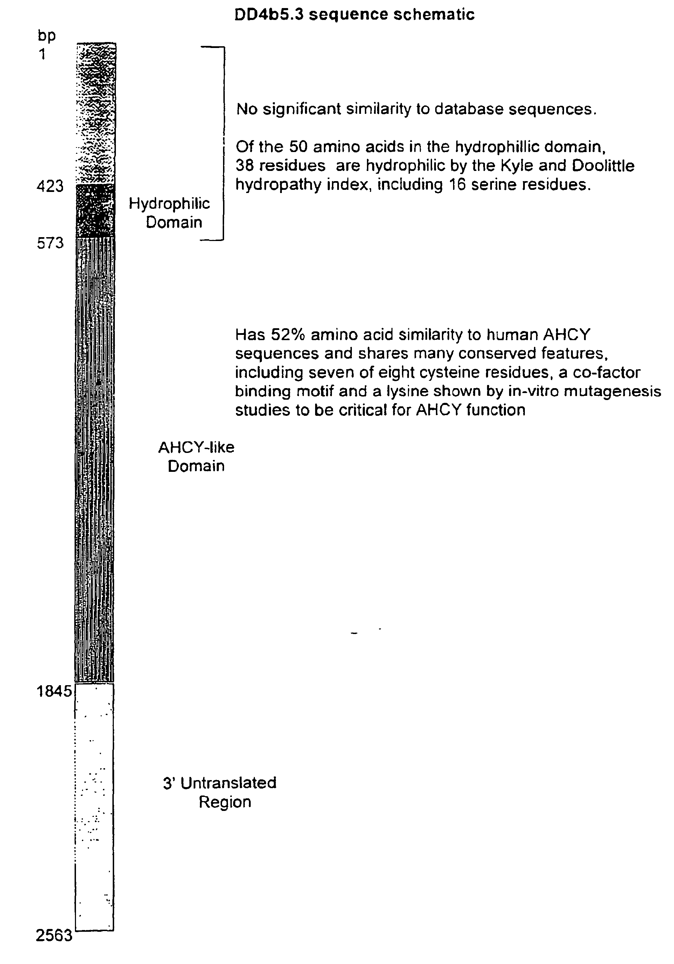

Translation of the cDNA sequence for DD4b5.3 revealed an open reading frame as indicated in Figure 1. Comparison of the putative DD4b5.3 amino acid sequence with the AHCY sequences of human', mouse (Genbank Accession no. L32836) and drosophila (Genbank Accession no. X95636) revealed expensive similarity to the AHCY sequences. (Figure 2). A further analysis of the structure is shown in Figure 3.

DD4b5.3 has a similar enzymatic function to AHCY based on significant identities in the AHCY related sequence. Thus DD4b5.3 has:
i) Cys¹¹³ and Cys¹⁹⁵ are conserved (numbering based on AHCY amino acid sequence (DD4b5.3 sequence)). Modification of these affects enzymatic function¹³. In contrast Cys⁴²¹ is non essential¹³ and this is substituted (Lys) in DD4b5.3.
ii) Lys⁴²⁶ identified as critical for AHCY function is preserved in DD4b5.3¹⁴. (Figure 2).
iii) Val¹⁷⁵ and Lys¹⁸⁶ predicted to be involved in Ade ring binding are conserved in DD4b5.3¹⁵. In contrast, Val³¹⁹ and Arg³²⁷ also predicted to be involved¹⁵ are not but the QVD sequence between them is, suggesting this may allow for an alternative specificity.
iv) Glu^{197 16} and Serine¹⁹⁸ which are also related to the active enzyme site are conserved in DD4b5.3.
v) The cofactor NAD binding site¹⁷ is highly conserved (Lys²¹⁴-Asp²³⁵) and the critical G-G--G binding site is present in DD4b5.3. (Figure 2)
vi) Eight Cys residues are thought to be involved in disulphide bonding. These contribute to maintaining the globular structure of the 4 subunits (~47,000) which make up the tetramer (~190,000) AHCY molecule in mammals. Seven of eight are preserved in DD4b5.3 amino acid sequence.

### Example 2

### Tissue Distribution

### Experimental 1

### Methods

### Northern blotting

Total RNA was extracted from L428 and U937 cell lines, electrophoresed on formaldehyde-denaturing agarose gels, transferred to nylon membranes and probed with 32P labeled DD clones.

### RT-PCR analysis

Based on sequence data, clone-specific oligonucleotide primers were synthesised to assess tissue distribution by RT-PCR. Two panels of haemopoetic cell populations were screened:
1) Cultured cell lines. Total RNA was extracted from cell lines, treated with RNAse-free DNAse I. and used for first stand cDNA synthesis. Aliquots of cDNA were used in PCRs with specific oligonucleotide primers.
2) Freshly isolated cells. First strand cDNA synthesis was performed directly on whole-cells isolated from human peripheral blood, based on established methods. Template consisted of 500 purified cells and the entire reaction was used in the subsequent PCR.

RT-PCR products were assessed by agarose gel electrophoresis. Identity of the products was confirmed by internal oligonucleotide hybridisation.

### Results

### Northern blot analysis

Northern blot analysis reveals DD4b.5.3 probe hybridises only to the L428 lanes.

### RT-PCR analysis of cell lines

Because L428 is a Hodgkin's disease cell line it is possible that DD products isolated from L428 may be expressed in other Hodgkin's cell lines. We assessed the expression of clone DD4b.5.3 in a cell line panel by RT-PCR (Table 1). The results indicate clone DD4b.5.3 is expressed showing a different pattern of expression across the different cell lines.

**Table 1**

| Cell line | DD4b.53 |
|---|---|
| L428 | +++ |
| act.U937 | + |
| U937 | + |
| Monomac | + |
| THP1 | + + |
| NB4 | + |
| KG1 | - |
| HDLM2 | +++ |
| KMH2 | + + |
| Raji | ++ |
| Mann | ++ |
| EBV-B cells | + |
| Jurkat | ++ |
| K562 | - |

| | |
|---|---|
| Relative band intensities: + + + strong, + + moderate, + weak, - no band visible. β2-microglobulin cDNA control PCR also performed. | |

### RT-PCR analysis of fresh cells

To assay the expression of the differential display clones in normal cell populations. RT-PCR was performed on freshly isolated cells. The results are summarised in Table 2.

**Table 2 Summary of RT-PCR results**

| RT-PCR Template | Phenotype | DD4b.5.3 |
|---|---|---|
| Fresh blood DC | DR⁺, Mix⁻ | + |
| Cultured Blood DC | DR⁻, CMRF44⁻ | ++ |
| Fresh Blood Monocytes | CD14⁻ | - |
| Cultured Blood Monocytes | CD14⁺ | |
| Fresh B Blood Lymphocytes | CD19⁻ | - |
| Cultured B Blood Lymphocytes | CD19⁻ | - |
| Blood T Lymphocytes | CD3⁻ | - |
| NK cells | CD16⁺, CD57⁻ | - |

| | | |
|---|---|---|
| 1 Semi-Nested PCR | | |
| Relative band intensities compared by ethidium bromide staining: ++ moderate, + weak, +/- very weak, - no product visible. | | |

### Experimental 2

### i) Cell lines

Northern blot analysis using RNA obtained from cell lines revealed specific hybridisation to a L428 transcript which was not detected in U937 (data not shown)

### ii) Leucocyte Populations

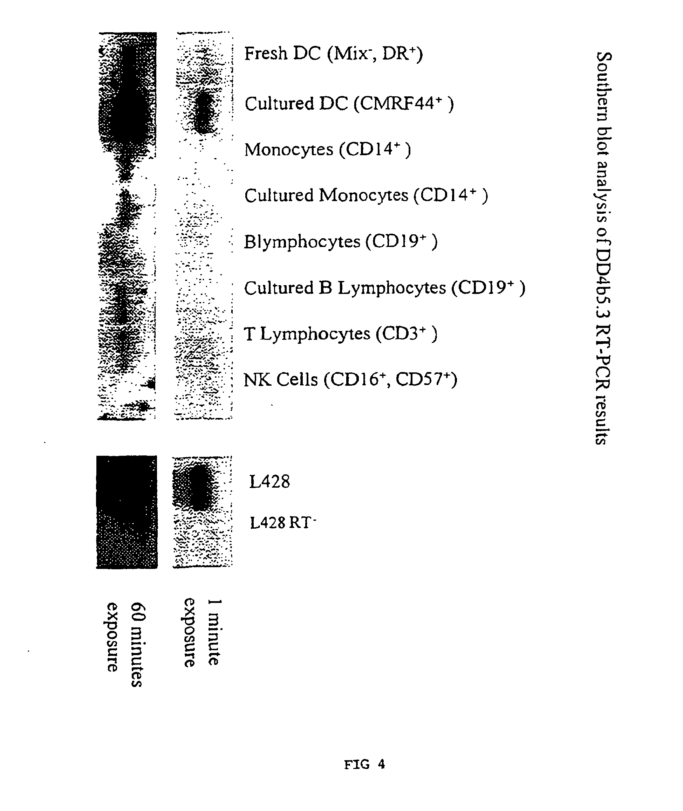

Semi-quantitative RT-PCR was performed on cDNA template derived from normal leukocyte cell populations using specific oligonucleotide primers. The cDNA equivalent from 125 cells was used in each reaction (Figure 4). To confirm the specificity of the DD4b5.3 RT-PCR (nucleotides 529-944), the products (416 bp) were blotted onto nylon membrane and probed with an internal digoxigenin (Boehringer Manheim) labelled oligonucleotide. Control β2-microglobulin RT-PCR analysis confirmed the template integrity.

Abundant DD4b5.3 message was observed after 1 min exposure, in activated DC (lane 2), and fresh DC (lane 1). Minimal expression in some other leukocyte populations was noted, but only after prolonged 60 min exposure.

### iii) DC Lineage

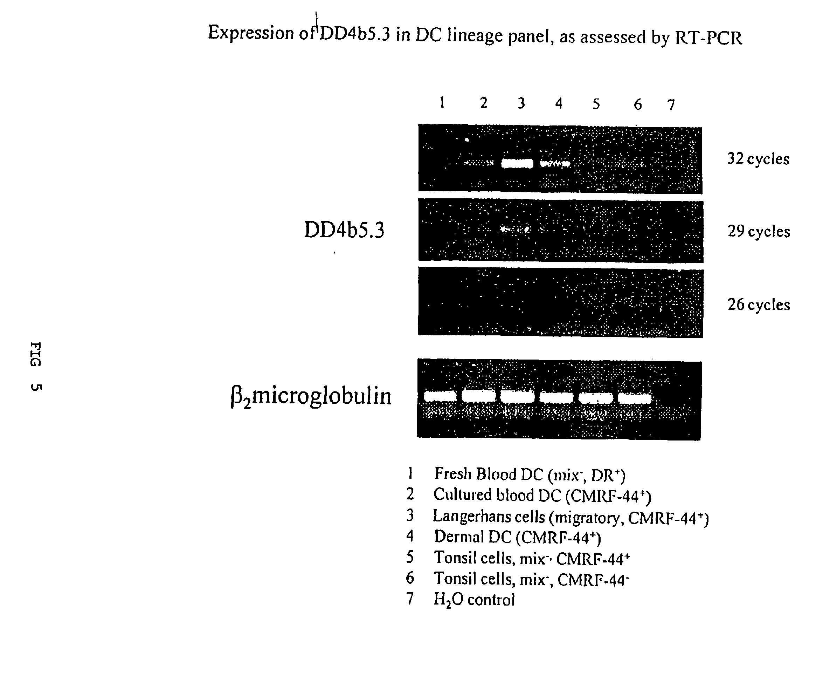

RT-PCR was performed using cDNA template from a range of different DC populations representing a putative DC differentiation/ activation pathway. (Figure 5). Using a range of cycle numbers an estimate of DD4b5.3 mRNA context in each type of DC preparation was obtained. Increased levels of DD4b5.3 mRNA were associated with the differentiation/ activation of DC derived from the blood and non-lymphoid tissues but little DD4b5.3 mRNA was detected in "mature" tonsil DC.

### Conclusion

The enzyme of the invention has a dendritic-cell-restricted expression pattern.

### INDUSTRIAL APPLICATION

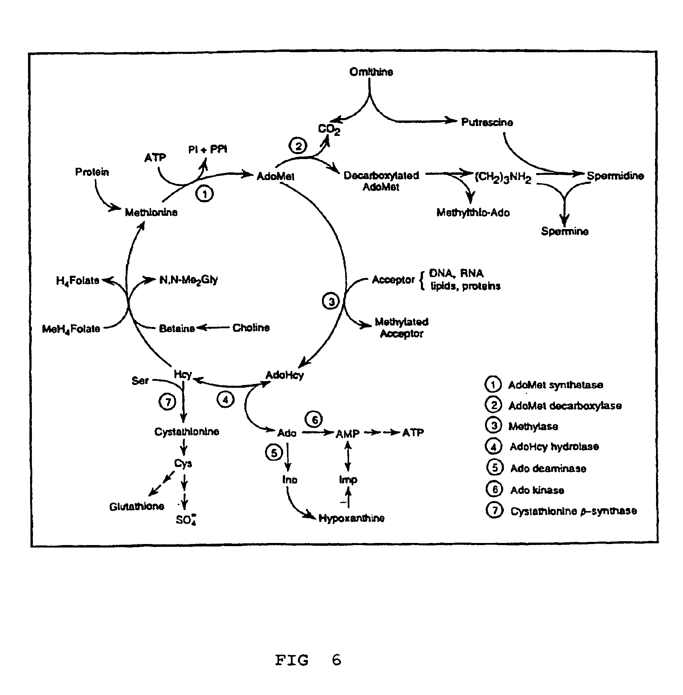

The significant amino acid identity of DD4b5.3 with human AHCY suggests that it has a related enzymatic function (ie. is of the AHCY-type) (see enzyme.4 in Figure 6).
As turnover of (AdoMet) varies between tissues it seems probable that different cell types may express different AHCY like enzymes for the pathways illustrated in Figure 6 or other pathways. DD4b5.3 may have such a function in DC. Its relatively selective expression in DC and its upregulation at certain stages of DC differentiation/ activation suggest it may be critical to DC function.

The novel N terminal sequence of the enzyme may be involved in localising DD4b5.3 to a specific intracellular compartment or play a part in regulating its enzymatic function perhaps by acting as a regulatory domain and binding to other proteins.

The enzyme of the invention will therefore have a number of potential applications. These include in immunomodulation (ie. manipulation of the immune response of a host). The enzyme will also have application in a screening programme to identify substances (usually compounds) which have either a stimulatory or inhibitory effect on its activity. More preferably, the program will be directed to determining the immunosuppressive potential of a test compound with reference to the ability of the compound to inhibit enzyme activity.

In this method, procedures identical or analogous to those outlined in Wolos *et al., Journal of Immunology* (1993), *supra* can be employed. Other conventional procedures for screening the effect of a test compound on enzyme activity can also be used.

Compounds identified in this way will have numerous potential applications.

AHCY itself can be inhibited by many nucleosides^{2.6-9} The compound MDL 28,842 has been reported to be immunosuppressive. It inhibited Con A and PWM induced proliferation of human peripheral blood mononuclear cells¹⁰. MDL 28.842 inhibited the Con A induced mouse T lymphocyte proliferation but not LPS induced B cell proliferation and reduced T lymphocyte IL-2 and IL-2R production¹⁰. *In vivo,* MDL 28,842 inhibited the mouse antibody response to the T lymphocyte dependent antigen ovalbumin¹⁰. The same compound prevented collagen induced arthritis in mice¹¹. Administration at 5mg/kg/day ip for 6 days prolonged skin graft survival to 12.2 days compared to 8.7 days in controls, a superior result to that obtained with Cyclosporin A at 5mg/kg/day¹². It may also inhibit heart allograft rejection in rats¹².

Equivalent applications are likely for compounds which inhibit the enzyme of the invention.

Blocking or inhibiting the activity of enzyme DD4b5.3 also gives rise to the following potential applications:
- Inhibition of DD4b5.3 may block DC function (eg: migration, antigen uptake, antigen processing and presentation or costimulation of T lymphoctyes). This may be profoundly immunosuppressive. DD4b5.3 can be expressed as a protein¹⁷ and nucleoside analogues or other compounds designed which inhibit its function. A precedent for selective functional inhibition is illustrated by the use of the AHCY inhibitory compound C3 ado which inhibits macrophage IL-1 synthesis but not T lymphocyte proliferation¹⁸.
   The identification of safe agents for *in vivo* use (note similar compounds have-been given to animals without major side-effects), which have a selective inhibitory effect on DC may allow the design of an important new immunosuppressive drug. This would have applications in all forms of transplantation and auto-immune disease.
- It is (theoretically) possible that blocking DD4b5.3 enzymatic function may have the reverse function, ie: boost their functional capabilities. As such, DD4b5.3 compounds might have immunostimulatory properties for vaccination eg. against infectious disease or for the immunotherapy of cancer.
- It is also conceivable that blocking DD4b5.3 might modify DC growth and differentiation facilitating the collection of DC for therapeutic use.

The enzyme of the invention can also be used to generate antibodies for example by the method of Kohler and Milstein (Kohler, G. and Milstein, C., Nature 256 495-497 (1975). Due to the restricted expression of the enzyme of the invention, such antibodies will have potential utility in the identification of DC in cytological preparations and tissue sections. The abnormal cells in Hodgkins disease have many features similar to DC. The enzyme is expressed in HD cell lines and the identification of the abnormal cells (Reed Sternberg cells and the related Hodgkins cells) which express the enzyme will be of value in the diagnosis of disorders such as Hodgkins disease²⁰.

The DNA sequence of the invention or any part thereof can also be used as a probe to discriminate for abnormal HD cells by in situ hybridisation. The use of nucleic acid probes is standard for example as described in Maniatis et al., "Molecular Cloning: A Laboratory Manual". Cold Spring Harbour (1982).

Other applications include the following:
- The DD4b5.3 sequence can be used to design anti -sense (anti-mRNA) reagents to suppress DC function, achieving the possible outcomes described above.
- The novel amino acid sequence towards the N terminus of the AHCY-like sequence may be used to target other compounds into the appropriate intracellular site to modify molecular functions, eg: to target other inhibitors of DD4b5.3.
- The identification of a novel AHCY-like sequence with additional N terminal sequence suggests there may be an extended family of such AHCY-like enzymes. The cDNA sequence of DD4b5.3 could be used to screen for other members of this family by a range of standard molecular biology techniques.

Those persons skilled in the art will of course appreciate that the above description is provided by way of example only and that the invention is not limited thereto.
1. Coulter-Karis DE, Hershfield MS: Sequence of full length cDNA for human S-adenosylhomocysteine hydrolase. Ann Hum Gen 53:169, 1989
2. Chiang PK, Gordon RK, Tal J. Zeng CG, Doctor BP, Pardhasaradhi K. McCann PP: S-Adenosylmethionine and methylation. FASEB 10:471. 1996
3. Borchardt RT: S-adenosyl-L-methionine-dependent macromolecule methyltransferases: potential targets for the destign of chemotherapeutic agents. J Med Chem 23:347, 1980
4. Chiang PK: S-adenosylhomocysteine hydrolase as a pharmacological target for the inhibition of transmethylation. Adv Exp Med Biol 165:199, 1984
5. Filgueira L, Zuber M, Juretic A, Luscher U, Caetano V, Harder F, Garotta G, Heberer M, Spagnoli GC: Differential effects of interleukin-2 and CD3 triggering on cytokine gene transcription and secretion in cultured tumor infiltrating lymphocytes. Cell Immunol 150:205, 1993
6. Wnuk SF, Yuan CS, Borchardt RG, Balzarini J, DeClercq E, Robins MJ: Nucleic acid related compound.84. Synthesis of 6'-(E and Z)-halohomovinyl derivatives of adenosine, inactivation of S-adenosyl-L-homocysteine hydrolase, and correlation of anticancer and antiviral potencies with enzyme inhibition. J Med Chem 37:3579, 1994
7. Liu S, Wolfe MS. Borchardt RT: Rational approaches to the design of antiviral agents based on S-adenosyl-L-homocysteine hydrolase as a molecular target. Antiviral Research 19:247, 1992
8. Paller AS. Arnsmeier SL, Clark SH, Mirkin BL: Z-4,5'-didehydro-5'-fluoroadenosine (MDL 28.842), an irreversible inhibitor of S-adenosylhomocysteine hydrolase, suppresses proliferation of cultured keratinocytes and squamous carcinoma cell lines (published erratum appears in Cancer Res 1994 Jul 15:54(14):3952). Cancer Res 53:6058, 1993
9. Schmidt JA. Bomford R, Gao X, Rhodes J: 3-Deazaadenosine - an inhibitor of interleukin 1 production by human peripheral blood monocytes. Int J Immunopharmacol 12:89, 1990
10. Wolos JA, Frondorf KA, Davis GF, Jarvi ET, McCarthy JR, Bowlin TL: Selective inhibition of T cell activation by an inhibitor of S-adenosyl-L-homocysteine hydrolase. J Immunol 150:3264, 1993
11. Wolos JA, Frondorf KA, Esser RE: Immunosuppression mediated by an inhibitor of S-Adenosyl-L-homocysteine hydrolase. J Immunol 151:526, 1993
12. Wolos JA. Frondorf KA, Babcock GF, Stripp SA, Bowlin TL: Immunomodulation by an inhibitor of S-adenosyl-L-homocysteine hydrolase: inhibition of in vitro and in vivo allogeneic responses. Cell Immunol 149:402. 1993
13. Yuan CS, Ault-Riche DB. Borchardt RT: Chemical modification and site-directed mutagenesis of cysteine residues in human placental S-adenosylhomocysteine hydrolase. J Biol Chem 271:28009, 1996
14. Ault-Riche DB, Yuan CS, Borchardt RT: A single mutation of lysine 426 of human placental S-adenosylhomocysteine hydrolase inactivates the enzyme. J Biol Chem 269:31472, 1994
15. German DC, Bloch CA, Kredich NM: Measurements of S-adenosylmethionine and L-homocysteine metabolism in cultured human lymphoid cells. Journal of Biological Chemistry 258:10997. 1983
16. Gupta RA. Yuan C, Ault-Riche DB, Borchardt RT: unknown. Arch Biochem Biophys 319:365, 1995
17. Ogawa H. Gomi T, Mueckler MM. Fujioka M. Backlund PSJ, Aksamit RR. Unson CG, Cantoni GL: Amino acid sequence of S-adenosyl-L-homocysteine hydrolase from rat liver as derived from the cDNA sequence. Proc Natl Acad Sci U S A 84:719, 1987
18. Yuan L, Kuramitsu Y, Li Y, Kobayashi M, Hosokawa M: Restoration of interleukin-2 production in tumor-bearingrats through reducing tumor-derived transforming growth factor β by treatment with bleomycin. Cancer Immunol Immunother 41:355, 1995
19. Pike MC, DeMeester CA: Inhibition of phosphoinosotide metabolism in human polymorphonuclear leukocytes by S-adenosylhomocysteine. Journal of Biological Chemistry 263:3592. 1988
20. Sorg UR, Morse TM, Patton WN, Hock BD, Angus HB, Robinson BA, Colls BM, Hart DNJ: Hodgkin's cells express CD83, a dendritic cell lineage associated marker. Pathology 29:294, 1997

## Claims

1. An enzyme having AHCY-type activity which includes amino acids 177 to 314 of the amino acid sequence of Figure 1.

2. An enzyme according to claim 1 which comprises amino acids 183 to 614 of the amino acid sequence of Figure 1.

3. An enzyme according to claim 1 which comprises amino acids 1 to 614 of the amino acid sequence of Figure 1.

4. An isolated DNA sequence comprising a nucleotide sequence selected from the group consisting of:
(a) a sequence which encodes an enzyme according to claim 1;
(b) a sequence which is a complement of a sequence (a);
(c) a sequence which is a reverse complement of a sequence (a); and
(d) a sequence which is a reverse sequence of a sequence (a).

5. A DNA sequence according to claim 4 in which sequence (a) comprises nucleotides 529 to 945 of the Figure 1 sequence.

6. A DNA sequence according to claim 4 in which sequence (a) comprises nucleotides 549 to 1844 of the Figure 1 sequence.

7. A DNA sequence according to claim 4 in which sequence (a) comprises nucleotides 1 to 1844 of the Figure 1 sequence.

8. A DNA construct comprising a DNA sequence according to any one of claims 4 to 7.

9. A DNA construct comprising, in the 5'-3' direction:
(a) a gene promoter sequence;
(b) an open reading frame coding for an enzyme according to claim 1; and
(c) a gene termination sequence.

10. A DNA construct according to claim 9 wherein the open reading frame is in a sense orientation.

11. A DNA construct according to claim 9 wherein the open reading frame is in an anti-sense orientation.

12. A DNA construct according to any one of claims 8 to 11, for use in modulating the activity of an enzyme according to claim in a patient.

13. Use of a DNA construct according to any one of claims 8 to 11 in the manufacture of a medicament for modulating the activity of an enzyme according to claim 1 in a patient.

14. A DNA construct according to claim 10, for use in amplifying the activity of an enzyme according to claim 1 in a patient.

15. Use of a DNA construct according to claim 10, in the manufacture of a medicament for amplifying the activity of an enzyme according to claim 1 in a patient.

16. A DNA construct according to claim 11, for use in suppressing the activity of an enzyme according to claim 1 in a patient.

17. Use of a DNA construct according to claim 11, in the manufacture of a medicament for suppressing the activity of an enzyme according to claim 1 in a patient.

18. A method of determining the modulatory potential of a compound on an enzyme according to claim 1 which comprises the step of determining the ability of said compound to modulate the activity of said enzyme.

19. An antibody which binds an enzyme according to claim 1.

## Patentansprüche

1. Enzym mit einer Aktivität vom AHCY-Typ, das die Aminosäuren 117 bis 314 der Aminosäuresequenz von Figur 1 beinhaltet.

2. Enzym nach Anspruch 1, das die Aminosäuren 183 bis 614 der Aminosäuresequenz von Figur 1 umfasst.

3. Enzym nach Anspruch 1, das die Aminosäuren 1 bis 614 der Aminosäuresequenz von Figur 1 umfasst.

4. Isolierte DNA-Sequenz, die eine Nukleotidsequenz umfasst, die aus der Gruppe ausgewählt ist, die besteht aus:
(a) einer Sequenz, die für ein Enzym gemäß Anspruch 1 kodiert;
(b) einer Sequenz, die ein Komplement einer Sequenz (a) ist;
(c) einer Sequenz, die ein reverses Komplement einer Sequenz (a) ist; und
(d) einer Sequenz, die eine reverse Sequenz einer Sequenz (a) ist.

5. DNA-Sequenz gemäß Anspruch 4, wobei die Sequenz (a) die Nukleotide 529 bis 945 der Sequenz von Figur 1 umfasst.

6. DNA-Sequenz gemäß Anspruch 4, wobei die Sequenz (a) die Nukleotide 549 bis 1844 der Sequenz von Figur 1 umfasst.

7. DNA-Sequenz gemäß Anspruch 4, wobei die Sequenz (a) die Nukleotide 1 bis 1844 der Sequenz von Figur 1 umfasst.

8. DNA-Konstrukt, das eine DNA-Sequenz gemäß einem der Ansprüche 4 bis 7 umfasst.

9. DNA-Konstrukt, das in 5'-3'-Richtung umfasst:
(a) eine Gen-Promotor-Sequenz;
(b) ein offenes Leseraster, das für ein Enzym gemäß Anspruch 1 kodiert; und
(c) eine Gen-Terminations-Sequenz.

10. DNA-Konstrukt nach Anspruch 9, wobei das offene Leseraster in einer Sense-Orientierung vorliegt.

11. DNA-Konstrukt nach Anspruch 9, wobei das offene Leseraster in einer Antisense-Orientierung vorliegt.

12. DNA-Konstrukt nach einem der Ansprüche 8 bis 11 zur Verwendung zur Modulation der Aktivität eines Enzyms gemäß Anspruch 1 bei einem Patienten.

13. Verwendung eines DNA-Konstrukts nach einem der Ansprüche 8 bis 11 bei der Herstellung eines Medikaments zur Modulation der Aktivität eines Enzyms gemäß Anspruch 1 bei einem Patienten.

14. DNA-Konstrukt nach Anspruch 10 zur Verwendung bei der Verstärkung der Aktivität eines Enzyms gemäß Anspruch 1 bei einem Patienten.

15. Verwendung eines DNA-Konstrukts nach Anspruch 10 bei der Herstellung eines Medikaments zur Verstärkung der Aktivität eines Enzyms gemäß Anspruch 1 bei einem Patienten.

16. DNA-Konstrukt nach Anspruch 11 zur Verwendung bei der Suppression der Aktivität eines Enzyms nach Anspruch 1 bei einem Patienten.

17. Verwendung eines DNA-Konstrukts nach Anspruch 11 bei der Herstellung eines Medikaments zur Suppression der Aktivität eines Enzyms gemäß Anspruch 1 bei einem Patienten.

18. Verfahren zur Bestimmung des modulatorischen Potentials einer Verbindung auf ein Enzym gemäß Anspruch 1, das die Stufe der Bestimmung der Fähigkeit der Verbindung, die Aktivität dieses Enzyms zu modulieren, umfasst.

19. Antikörper, der ein Enzym gemäß Anspruch 1 bindet.

## Revendications

1. Enzyme ayant une activité de type AHCY qui inclut les acides aminés 177 à 314 de la séquence d'acides aminés de la figure 1.

2. Enzyme selon la revendication 1, qui comprend les acides aminés 183 à 614 de la séquence d'acides aminés de la figure 1.

3. Enzyme selon la revendication 1, qui comprend les acides aminés 1 à 614 de la séquence d'acides aminés de la figure 1.

4. Séquence d'ADN isolée comprenant une séquence nucléotidique choisie dans le groupe constitué par :
(a) une séquence qui code une enzyme selon la revendication 1;
(b) une séquence qui est un complément de la séquence (a) ;
(c) une séquence qui est un complément inverse d'une séquence (a) ; et
(d) une séquence qui est une séquence inverse d'une séquence (a).

5. Séquence d'ADN selon la revendication 4, dans laquelle la séquence (a) comprend les nucléotides 529 à 945 de la séquence de la figure 1.

6. Séquence d'ADN selon la revendication 4, dans laquelle la séquence (a) comprend les nucléotides 549 à 1844 de la séquence de la figure 1.

7. Séquence d'ADN selon la revendication 4, dans laquelle la séquence (a) comprend les nucléotides 1 à 1 844 de la séquence de la figure 1.

8. Construction d'ADN comprenant une séquence d'ADN selon l'une quelconque des revendications 4 à 7.

9. Construction d'ADN comprenant, dans la direction 5' à 3' :
(a) une séquence de promoteur de gène ;
(b) un cadre ouvert de lecture codant une enzyme selon la revendication 1 ; et
(c) une séquence de terminaison d'un gène.

10. Construction d'ADN selon la revendication 9, dans lequel le cadre ouvert de lecture est dans une orientation sens.

11. Construction d'ADN selon la revendication 9, dans lequel le cadre ouvert de lecture est dans une orientation antisens.

12. Construction d'ADN selon l'une quelconque des revendications 8 à 11, pour une utilisation dans la modulation de l'activité d'une enzyme selon la revendication 1 chez un patient.

13. Utilisation d'une construction d'ADN selon l'une quelconque des revendications 8 à 11, dans la fabrication d'un médicament pour moduler l'activité d'une enzyme selon la revendication 1 chez un patient.

14. Construction d'ADN selon la revendication 10, pour une utilisation dans l'amplification d'activité d'une enzyme selon la revendication 1 chez un patient

15. Utilisation d'une construction d'ADN selon la revendication 10, dans la fabrication d'un médicament pour amplifier l'activité d'une enzyme selon la revendication 1 chez un patient.

16. Construction d'ADN selon la revendication 11, pour une utilisation dans la suppression de l'activité d'une enzyme selon la revendication 1 chez un patient.

17. Utilisation d'une construction d'ADN selon la revendication 11, dans la fabrication d'un médicament pour supprimer l'activité d'une enzyme selon la revendication 1 chez un patient

18. Procédé de détermination du potentiel de modulation d'un composé sur une enzyme selon la revendication 1, qui comprend l'étape consistant à déterminer la capacité dudit composé à moduler l'activité de ladite enzyme.

19. Anticorps qui se lie à une enzyme selon la revendication 1.


<110> Hart, Derek N J
<120> Enzyme having S-adenosyl-L-homocysteine hydrolase (ACHY) type activity
<130> 24303 MRB
<140> EP 97945109.3
   <141> 1997-10-06
<150> PCT/NZ97/00133
   <151> 1997-10-06
<150> NZ 299507
   <151> 1996-10-04
<160> 2
<170> PatentIn Ver. 2.1
<210> 1
   <211> 2563
   <212> DNA
   <213> Homo sapiens
<220>
   <221> CDS
   <222> (3)..(1897)
   <223> Open reading frame extends without a stop codon for the full 5' nucleotide sequence. The initiation codon has yet to be identified.
<400> 1
   gg cgc ggg cag gtc gga gct cgg agc tgc tgc ttc tgg ttc tct tgt      47
      Arg Gly Gln Val Gly Ala Arg Ser Cys Cys Phe Trp Phe Ser Cys
        1               5                  10                  15
   ggc cac cgt cgc tgt ccg gct gcc ttg ggc tgc cga aca gac aag gcg     95
   Gly His Arg Arg Cys Pro Ala Ala Leu Gly Cys Arg Thr Asp Lys Ala
                    20                  25                  30
   tgg gcc aca gca cct cag aag ccg acg cag ctc gac gca ggg gcc ggc    143
   Trp Ala Thr Ala Pro Gln Lys Pro Thr Gln Leu Asp Ala Gly Ala Gly
                35                  40                  45
   agg agg gtg ggc gat cgc gtg tcg gag ggc gcc gcg cgg gca ggc ggg    191
   Arg Arg Val Gly Asp Arg Val Ser Glu Gly Ala Ala Arg Ala Gly Gly
            50                  55                  60
   cgg gcg cca gag ggg gaa aga ggc ggg ggc ggc ggg tca gcc gct ggc    239
   Arg Ala Pro Glu Gly Glu Arg Gly Gly Gly Gly Gly Ser Ala Ala Gly
        65                  70                  75
   cgg gcc ggc ggg gga atg tcg atg cct gac gcg atg ccg ctg ccc ggg    287
   Arg Ala Gly Gly Gly Met Ser Met Pro Asp Ala Met Pro Leu Pro Gly
    80                  85                  90                  95
   gtc ggg gag gag ctg aag cag gcc aag gag atc gag gac gcc gag aag    335
   Val Gly Glu Glu Leu Lys Gln Ala Lys Glu Ile Glu Asp Ala Glu Lys
                   100                 105                 110
   tac tcc ttc atg gcc acc gtc acc aag gcg ccc aag aag caa atc cag     383
   Tyr Ser Phe Met Ala Thr Val Thr Lys Ala Pro Lys Lys Gln Ile Gln
               115                 120                 125
   ttt gct gat gac atg cag gag ttc acc aaa ttc ccc acc aaa act ggc     431
   Phe Ala Asp Asp Met Gln Glu Phe Thr Lys Phe Pro Thr Lys Thr Gly
           130                 135                 140
   cga aga tct ttg tct cgc tcg atc tca cag tcc tcc act gac agc tac     479
   Arg Arg Ser Leu Ser Arg Ser Ile Ser Gln Ser Ser Thr Asp Ser Tyr
       145                 150                 155
   agt tca gct gca tcc tac aca gat agc tct gat gat gag gtt tct ccc     527
   Ser Ser Ala Ala Ser Tyr Thr Asp Ser Ser Asp Asp Glu Val Ser Pro
   160                 165                 170                 175
   cga gag aag cag caa acc aac tcc aag ggc agc agc aat ttc tgt gtg     575
   Arg Glu Lys Gln Gln Thr Asn Ser Lys Gly Ser Ser Asn Phe Cys Val
                   180                 185                 190
   aag aac atc aag cag gca gaa ttt gga cgc cgg gag att gag att gca     623
   Lys Asn Ile Lys Gln Ala Glu Phe Gly Arg Arg Glu Ile Glu Ile Ala
               195                 200                 205
   gag caa gac atg tct gct ctg att tca ctc agg aaa cgt gct cag ggg     671
   Glu Gln Asp Met Ser Ala Leu Ile Ser Leu Arg Lys Arg Ala Gln Gly
           210                 215                 220
   gag aag ccc ttg gct ggt gct aaa ata gtg ggc tgt aca cac atc aca     719
   Glu Lys Pro Leu Ala Gly Ala Lys Ile Val Gly Cys Thr His Ile Thr
       225                 230                 235
   gcc cag aca gcg gtg ttg att gag aca ctc tgt gcc ctg ggg gct cag     767
   Ala Gln Thr Ala Val Leu Ile Glu Thr Leu Cys Ala Leu Gly Ala Gln
   240                 245                 250                 255
   tgc cgc tgg tct gct tgt aac atc tac tca act cag aat gaa gta gct     815
   Cys Arg Trp Ser Ala Cys Asn Ile Tyr Ser Thr Gln Asn Glu Val Ala
                   260                 265                 270
   gca gca ctg gct gag gct gga gtt gca gtg ttc gct tgg aag ggc gag     863
   Ala Ala Leu Ala Glu Ala Gly Val Ala Val Phe Ala Trp Lys Gly Glu
               275                 280                 285
   tca gaa gat gac ttc tgg tgg tgt att gac cgc tgt gtg aac atg gat     911
   Ser Glu Asp Asp Phe Trp Trp Cys Ile Asp Arg Cys Val Asn Met Asp
           290                 295                 300
   ggg tgg cag gcc aac atg atc ctg gat gat ggg gga gac tta acc cac     959
   Gly Trp Gln Ala Asn Met Ile Leu Asp Asp Gly Gly Asp Leu Thr His
       305                 310                 315
   tgg gtt tat aag aag tat cca aac gtg ttt aag aag atc cga ggc att    1007
   Trp Val Tyr Lys Lys Tyr Pro Asn Val Phe Lys Lys Ile Arg Gly Ile
   320                 325                 330                 335
   gtg gaa gag agc gtg act ggt gtt cac agg ctg tat cag ctc tcc aaa    1055
   Val Glu Glu Ser Val Thr Gly Val His Arg Leu Tyr Gln Leu Ser Lys
                   340                 345                 350
   gct ggg aag ctc tgt gtt ccg gcc atg aac gtc aat gat tct gtt acc    1103
   Ala Gly Lys Leu Cys Val Pro Ala Met Asn Val Asn Asp Ser Val Thr
               355                 360                 365
   aaa cag aag ttt gat aac ttg tac tgc tgc cga gaa tcc att ttg gat    1151
   Lys Gln Lys Phe Asp Asn Leu Tyr Cys Cys Arg Glu Ser Ile Leu Asp
           370                 375                 380
   ggc ctg aag agg acc aca gat gtg atg ttt ggt ggg aaa caa gtg gtg    1199
   Gly Leu Lys Arg Thr Thr Asp Val Met Phe Gly Gly Lys Gln Val Val
       385                 390                 395
   gtg tgt ggc tat ggt gag gta ggc aag ggc tgc tgt gct gct ctc aaa    1247
   Val Cys Gly Tyr Gly Glu Val Gly Lys Gly Cys Cys Ala Ala Leu Lys
   400                 405                 410                 415
   gct ctt gga gca att gtc tac att acc gaa atc gac ccc atc tgt gct    1295
   Ala Leu Gly Ala Ile Val Tyr Ile Thr Glu Ile Asp Pro Ile Cys Ala
                   420                 425                 430
   ctg cag gcc tgc atg gat ggg ttc agg gtg gta aag cta aat gaa gtc    1343
   Leu Gln Ala Cys Met Asp Gly Phe Arg Val Val Lys Leu Asn Glu Val
               435                 440                 445
   atc cgg caa gtc gat gtc gta ata act tgc aca gga aat aag aat gta    1391
   Ile Arg Gln Val Asp Val Val Ile Thr Cys Thr Gly Asn Lys Asn Val
           450                 455                 460
   gtg aca cgg gag cac ttg gat cgc atg aaa aac agt tgt atc gta tgc    1439
   Val Thr Arg Glu His Leu Asp Arg Met Lys Asn Ser Cys Ile Val Cys
       465                 470                 475
   aat atg ggc cac tcc aac aca gaa atc gat gtg acc agc ctc cgc act    1487
   Asn Met Gly His Ser Asn Thr Glu Ile Asp Val Thr Ser Leu Arg Thr
   480                 485                 490                 495
   ccg gag ctg acg tgg gag cga gta cgt tct cag gtg gac cat gtc atc    1535
   Pro Glu Leu Thr Trp Glu Arg Val Arg Ser Gln Val Asp His Val Ile
                   500                 505                 510
   tgg cca gat ggc aaa cga gtt gtc ctc ctg gca gag ggt cgt cta ctc    1583
   Trp Pro Asp Gly Lys Arg Val Val Leu Leu Ala Glu Gly Arg Leu Leu
               515                 520                 525
   aat ttg agc tgc tcc aca gtt ccc acc ttt gtt ctg tcc atc aca gcc    1631
   Asn Leu Ser Cys Ser Thr Val Pro Thr Phe Val Leu Ser Ile Thr Ala
           530                 535                 540
   aca aca cag gct ttg gca ctg ata gaa ctc tat aat gca ccc gag ggg    1679
   Thr Thr Gln Ala Leu Ala Leu Ile Glu Leu Tyr Asn Ala Pro Glu Gly
       545                 550                 555
   cga tac aag cag gat gtg tac ttg ctt cct aag aaa atg gat gaa tac    1727
   Arg Tyr Lys Gln Asp Val Tyr Leu Leu Pro Lys Lys Met Asp Glu Tyr
   560                 565                 570                 575
   gtt gcc agc ttg cat ctg cca tca ttt gat gcc cac ctt aca gag ctg    1775
   Val Ala Ser Leu His Leu Pro Ser Phe Asp Ala His Leu Thr Glu Leu
                   580                 585                 590
   aca gat gac caa gca aaa tat ctg gga ctc aac aaa aat ggg cca ttc   1823
   Thr Asp Asp Gln Ala Lys Tyr Leu Gly Leu Asn Lys Asn Gly Pro Phe
               595                 600                 605
   aaa cct aat tat tac aga tac taa tggaccatac taccaaggac cagtccacct  1877
   Lys Pro Asn Tyr Tyr Arg Tyr
           610                 615
   gaaccacaca ctctaaagaa atatttttta agataacttt tattttcttc ttactccttt 1937
   cctcttgatt tttttcctat aatttcattc ttgttttttc atctcattat ccaagttctg 1997
   cagaccacac aggaacttgc ttcatggctc tttagatgaa atagaagttc agggtccctc 2057
   actctagtca ctaaagaagg attttactcc cccagcccag aaaggtgatt cttctcttta 2117
   ccatttctgg ggactttagt cttaattagg taccttatta acaggaaatg ctaaggtacc 2177
   ttctctgtgg aacaatctgc aatgtctaaa tcgccttaaa agagcccatt tcttagctgc 2237
   tgaaatcagt gctctttcac ttcttcagag aagcagggat ggtacctacc cggcaggtag 2297
   gttagatgtg ggtggtgcat gttaatttcc cttagaagtt ccaagccctg tttcctgcgt 2357
   aaaggtggta tgtccagttc agagatgtgt ataatgagca tggcttgtta agatcaggag 2417
   gcccacttgg atttatagta tagcccttcc tccactccca ccagacttgc tcatttttcg 2477
   agtttttaac tagactacac tctatttgag tttaattttg tcctctagga tttatttctg 2537
   ttgtccaaaa aaaaaaaaaa aaaaaa                                      2563
<210> 2
   <211> 614
   <212> PRT
   <213> Homo sapiens
<400> 2
   Arg Gly Gln Val Gly Ala Arg Ser Cys Cys Phe Trp Phe Ser Cys Gly
    1               5                  10                  15
   His Arg Arg Cys Pro Ala Ala Leu Gly Cys Arg Thr Asp Lys Ala Trp
               20                  25                  30
   Ala Thr Ala Pro Gln Lys Pro Thr Gln Leu Asp Ala Gly Ala Gly Arg
           35                  40                  45
   Arg Val Gly Asp Arg Val Ser Glu Gly Ala Ala Arg Ala Gly Gly Arg
       50                  55                  60
   Ala Pro Glu Gly Glu Arg Gly Gly Gly Gly Gly Ser Ala Ala Gly Arg
   65                  70                  75                  80
   Ala Gly Gly Gly Met Ser Met Pro Asp Ala Met Pro Leu Pro Gly Val
                   85                  90                  95
   Gly Glu Glu Leu Lys Gln Ala Lys Glu Ile Glu Asp Ala Glu Lys Tyr
               100                 105                 110
   Ser Phe Met Ala Thr Val Thr Lys Ala Pro Lys Lys Gln Ile Gln Phe
               115                 120                 125
   Ala Asp Asp Met Gln Glu Phe Thr Lys Phe Pro Thr Lys Thr Gly Arg
       130                 135                 140
   Arg Ser Leu Ser Arg Ser Ile Ser Gln Ser Ser Thr Asp Ser Tyr Ser
   145                 150                 155                 160
   Ser Ala Ala Ser Tyr Thr Asp Ser Ser Asp Asp Glu Val Ser Pro Arg
                   165                 170                 175
   Glu Lys Gln Gln Thr Asn Ser Lys Gly Ser Ser Asn Phe Cys Val Lys
               180                 185                 190
   Asn Ile Lys Gln Ala Glu Phe Gly Arg Arg Glu Ile Glu Ile Ala Glu
           195                 200                 205
   Gln Asp Met Ser Ala Leu Ile Ser Leu Arg Lys Arg Ala Gln Gly Glu
       210                 215                 220
   Lys Pro Leu Ala Gly Ala Lys Ile Val Gly Cys Thr His Ile Thr Ala
   225                 230                 235                 240
   Gln Thr Ala Val Leu Ile Glu Thr Leu Cys Ala Leu Gly Ala Gln Cys
                   245                 250                 255
   Arg Trp Ser Ala Cys Asn Ile Tyr Ser Thr Gln Asn Glu Val Ala Ala
               260                 265                 270
   Ala Leu Ala Glu Ala Gly Val Ala Val Phe Ala Trp Lys Gly Glu Ser
           275                 280                 285
   Glu Asp Asp Phe Trp Trp Cys Ile Asp Arg Cys Val Asn Met Asp Gly
       290                 295                 300
   Trp Gln Ala Asn Met Ile Leu Asp Asp Gly Gly Asp Leu Thr His Trp
   305                 310                 315                 320
   Val Tyr Lys Lys Tyr Pro Asn Val Phe Lys Lys Ile Arg Gly Ile Val
                   325                 330                 335
   Glu Glu Ser Val Thr Gly Val His Arg Leu Tyr Gln Leu Ser Lys Ala
               340                 345                 350
   Gly Lys Leu Cys Val Pro Ala Met Asn Val Asn Asp Ser Val Thr Lys
           355                 360                 365
   Gln Lys Phe Asp Asn Leu Tyr Cys Cys Arg Glu Ser Ile Leu Asp Gly
       370                 375                 380
   Leu Lys Arg Thr Thr Asp Val Met Phe Gly Gly Lys Gln Val Val Val
   385                 390                 395                 400
   Cys Gly Tyr Gly Glu Val Gly Lys Gly Cys Cys Ala Ala Leu Lys Ala
                   405                 410                 415
   Leu Gly Ala Ile Val Tyr Ile Thr Glu Ile Asp Pro Ile Cys Ala Leu
               420                 425                 430
   Gln Ala Cys Met Asp Gly Phe Arg Val Val Lys Leu Asn Glu Val Ile
           435                 440                 445
   Arg Gln Val Asp Val Val Ile Thr Cys Thr Gly Asn Lys Asn Val Val
       450                 455                 460
   Thr Arg Glu His Leu Asp Arg Met Lys Asn Ser Cys Ile Val Cys Asn
   465                 470                 475                 480
   Met Gly His Ser Asn Thr Glu Ile Asp Val Thr Ser Leu Arg Thr Pro
                   485                 490                 495
   Glu Leu Thr Trp Glu Arg Val Arg Ser Gln Val Asp His Val Ile Trp
               500                 505                 510
   Pro Asp Gly Lys Arg Val Val Leu Leu Ala Glu Gly Arg Leu Leu Asn
           515                 520                 525
   Leu Ser Cys Ser Thr Val Pro Thr Phe Val Leu Ser Ile Thr Ala Thr
       530                 535                 540
   Thr Gln Ala Leu Ala Leu Ile Glu Leu Tyr Asn Ala Pro Glu Gly Arg
   545                 550                 555                 560
   Tyr Lys Gln Asp Val Tyr Leu Leu Pro Lys Lys Met Asp Glu Tyr Val
                   565                 570                 575
   Ala Ser Leu His Leu Pro Ser Phe Asp Ala His Leu Thr Glu Leu Thr
               580                 585                 590
   Asp Asp Gln Ala Lys Tyr Leu Gly Leu Asn Lys Asn Gly Pro Phe Lys
           595                 600                 605
   Pro Asn Tyr Tyr Arg Tyr
       610